# EUROPEAN PATENT SPECIFICATION

(11) **EP 2 296 714 B1**
(45) Date of publication and mention of the grant of the patent: **07.11.2018**
(21) Application number: 09745762.6
(22) Date of filing: 13.05.2009
(51) Int. Cl.: A61L 9/14, A61L 2/22

(54) **METHOD FOR DISINFECTING A SPACE**
VERFAHREN ZUR DESINFEKTION EINES RAUMS
PROCÉDÉ DE DÉSINFECTION D'UN ESPACE

(30) Priority: 13.05.2008 NL 2001574; 16.05.2008 NL 2001586; 29.10.2008 NL 1036129
(43) Date of publication of application: 23.03.2011
(73) Proprietor: Infection Control B.V., 3755 BS Eemnes (NL)
(72) Inventor: Plantinga, Paul, NL-1276 HD Huizen (NL); Alma, Sander, Albert, NL-1241 CR Kortenhoef (NL); Musters, Carolus, Borromeus, Henricus, NL-1251 BE Laren (NL); Timmerman, Cornelis, Pieter, NL-3583 VG Utrecht (NL); Klaassen, Frank, Olaf, NL-1241 XJ Kortenhoef (NL)
(74) Representative: van Kooij, Adriaan
(86) International application number: PCT/EP2009/055780
(87) International publication number: WO 2009/138430

(56) References cited:
- EP-A- 0 774 263
- EP-A- 1 759 715
- WO-A-2007/125100
- US-A- 5 173 258

## Description

The present invention relates to a method for disinfecting a space and/or the objects possibly present therein.

In, among others, the pharmaceutical, biotechnological and medical industries, and in health care institutions and in the agrarian sector the disinfecting of spaces, surfaces and the objects present therein, such as for instance medical equipment, is generally known and of very great importance. The number of micro-organisms, such as bacteria, spores, viruses, fungi) in the space and on the relevant objects is greatly reduced by disinfecting such spaces, such as for instance operating rooms and nursing wards. Nosocomial infections of patients and/or staff in hospitals can in this way be combated and prevented.

Traditional disinfection methods for such spaces consist of, among others, manual cleaning of surfaces using chemical disinfectants. When surfaces are disinfected however, only the surface accessible by hand is treated, and for instance not the air, and the locations in this space which cannot be reached by hand. Staff are moreover necessary for the purpose of manual disinfection, and the effectiveness of the disinfection depends, among other factors, on the person carrying out the disinfection and is not always the same.

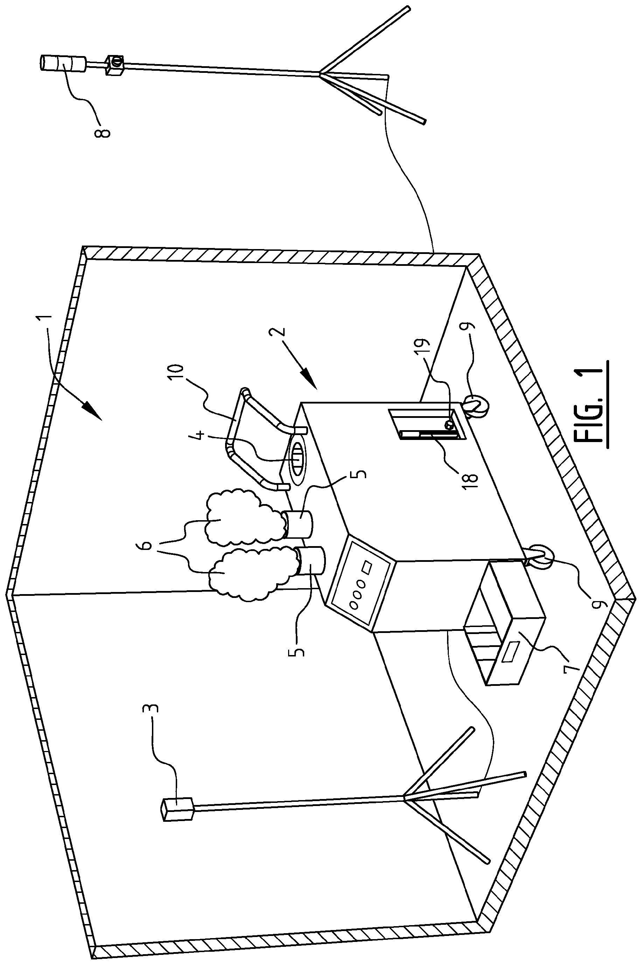

It is also known to make use of special spray devices with which a disinfecting liquid is sprayed into the space and onto the objects present therein. The known spray devices produce liquid droplets with a size of more than 20 pm, in general even between 80 and 200 µm (usually by atomization under pressure from a nozzle). Owing to the size and density of these droplets a so-called umbrella effect will occur during spraying, wherein a high concentration of the disinfecting liquid remains close to the spray device and the quantity of disinfectant in the air decreases very rapidly as the distance from the spray device increases. The space and the objects present therein will in this way not be disinfected uniformly because the disinfecting liquid will not reach some locations, or hardly so (see figure 1).

Despite the existing disinfection methods it occurs more and more frequently that during a stay in hospital patients are infected with pathogenic hospital bacteria, such as with, among others, the antibioticresistant MRSA bacteria.

Described in WO 2007/125100 are a method and device for effectively disinfecting spaces and/or the objects possibly present therein, which method comprises the following steps of:
(a) determining a first value of the relative humidity (RH) of the air in the space;
(b) atomizing a disinfecting liquid in the space until a predetermined second value of the relative humidity of the air in the space is reached, wherein the atomizing of the disinfecting liquid takes place by means of ultrasonic sound waves; and
(c) maintaining the relative air humidity for a predetermined time at the second value by means of atomizing the disinfecting liquid.

Although using this method the spaces and/or the objects present therein can be effectively disinfected, a so-called "tailing effect" is sometimes observed, i.e. an effect in which, after the first initial increase in the concentration of the disinfecting liquid, for instance hydrogen peroxide, in the air, this concentration gradually decreases in the course of time during the treatment despite continued atomizing of the disinfecting liquid in the space.

The present invention has for its object to provide a method for disinfecting spaces and/or the objects possibly present therein, with which the above described problems of the prior art methods are resolved.

This object is achieved by the present invention in that a method is provided which comprises the following steps of:
(a) determining a first value of the relative humidity of the air in the space;
(b) atomizing a disinfecting liquid in the space until a predetermined second value of the relative humidity of the air is reached in the space, and;
(c) maintaining the relative humidity of the air for a predetermined time at the second value by means of atomizing the disinfecting liquid,
wherein the method further comprises of: decreasing the relative humidity of the air in the space prior to and/or during atomizing of the disinfecting liquid.

Using the method according to the present invention the spaces and the objects possibly present therein are effectively disinfected, wherein an optimum reduction (> log 5) of the micro-organisms is achieved. The ambient temperature in the space here preferably lies between about 10° and about 40°C. It has been found that using the method according to the invention the tailing effect (a gradual decrease in the concentration of disinfecting liquid in the air during the treatment) does not occur, or hardly so. It has been found surprisingly according to the invention that by extracting moisture from the air, wherein it would be expected that the concentration of the atomized disinfecting liquid would also decrease, the concentration of the disinfecting liquid, such as for instance hydrogen peroxide, in the air in the space decreases less quickly than the air humidity. On the contrary, by simultaneously increasing the air humidity by atomizing disinfecting liquid in the space, the concentration of disinfecting liquid in the air becomes higher than without the step of dehumidifying the air. The moisture removed from the air still comprises hydrogen peroxide, although experiments have shown that the liquid condensed from the air contains less than 60% of the initial concentration (for instance a mere 2% instead of the initial concentration of 5%).

In a preferred embodiment of the invention the decrease in the humidity of the air in the space is started with a predetermined delay relative to starting of the atomization of the disinfecting liquid. The concentration of the disinfecting liquid can hereby first rise rapidly to a high peak value. The predetermined delay depends on the volume of the space to be treated. The delay preferably comprises 1-15 minutes, more preferably 5-10 minutes, for instance 10 minutes.

The decrease in the humidity of the air in the space during the treatment can take place continuously or at one or more determined points in time during the disinfection treatment. The decrease in the humidity of the air is preferably carried out continuously during atomizing of the disinfecting liquid. The above described tailing effect is herein avoided as far as possible.

Any suitable form of atomizing can be used in the method according to the invention. However, in a further preferred embodiment of the invention atomizing of the disinfecting liquid takes place by means of ultrasonic sound waves, preferably at a frequency of about 1.5 -2.8 MHz, more preferably at a frequency of about 1.5 - 2.0 MHz, more preferably about 1.7 MHz. Used for this purpose are ultrasonic elements, so-called piezoelectric ceramic discs. These ultrasonic elements are situated in the disinfecting liquid and are driven using an ultrasonic generator which sends current pulses to the elements at a frequency of for instance 1.7 MHz (1,700,000 vibrations per second). These current pulses cause the elements in the same frequency to expand and contract, whereby they are set into vibration, and the disinfecting liquid in which the elements are arranged also begins to vibrate. Because the disinfecting liquid is too slow to follow the movement of the ultrasonic elements, small vacuum bubbles are created in the liquid. These bubbles then implode under the influence of the surface tension of the liquid, a phenomenon known as cavitation. Very great forces can occur here, whereby the liquid is pushed out of the liquid surface in the form of very small droplets. These small droplets are then taken up into the air being guided over the bath with disinfecting liquid. Owing to the small dimensions and weight of the liquid droplets no condensation occurs.

As an alternative, atomizing of the disinfecting liquid can take place by passing the disinfecting liquid through a suitable nozzle, in particular an atomizing nozzle. Atomizing or nebulising a liquid using nozzles as such is well known and results in a suitable spray or mist of disinfecting liquid. Preferably the liquid is passed through the nozzle under pressure. Any suitable compressor can be used, for instance a combustion engine based compressor or an electric compressor.

In a further preferred embodiment of the invention atomizing of the disinfecting liquid comprises the sublimation of a disinfecting medium, wherein the disinfecting medium comprises the disinfecting liquid in the solid state. A rapid transition between the solid and the gas state by adding a suitable amount of heat results in an efficient gasification process which allows a homogeneous distribution of the disinfecting medium in the space. By the subsequent condensation of the disinfecting medium after gasification, the relative humidity in the space is increased. Preferably, said disinfecting medium comprises freeze dried disinfection liquid according to the invention.

According to the method of the present invention a first value of the relative humidity (RH) of the air in the space, i.e. the initial value, is first determined. A disinfection cycle is then started after a predetermined time. Any personnel that may be present must all leave the space before atomizing of the disinfecting liquid begins. When the disinfection cycle begins the disinfecting liquid is atomized until a predetermined second value of the relative humidity of the air in the space is reached, after which this RH is maintained at this value for a predetermined time by means of atomizing the disinfecting liquid. Prior to and/or during atomizing of the disinfecting liquid the relative humidity is once again decreased, i.e. the air in the space is dehumidified. Dehumidification of the air can be performed with standard means for dehumidifying the air in a space, such as for instance standard air-conditioning systems with internal recirculation, glycol coolers and the like, wherein it is important that the moisture is removed from the air and stored/discharged in liquid (condensed) form. In particular in combination with atomizing by sublimation, it can be advantageously to solidify the moisture from the air, for instance using freeze drying techniques. The air dehumidifying process can take place at any location in the space, even in the atomizing column. The atomizing means, for instance the ultrasonic generator, are switched off at the end of a disinfection cycle and no more disinfecting liquid is atomized. It is subsequently possible to enter the space again after a determined period of time.

In a further preferred embodiment of the invention the second value of the relative humidity is higher than the initial value measured in step (a). The second value of the relative humidity is preferably at least 10% higher, more preferably at least 15%, most preferably at least 20% higher than the first value. In a particular preferred embodiment of the invention the second value of the relative humidity is about 25% higher than the initial value. Increasing the relative humidity in the space by atomizing the disinfecting liquid achieves that the produced mist with the disinfectant dissolved therein is taken up into the air that is present. Because the air humidity is the same in the whole space (homogeneous distribution), the atomized disinfectant will also spread homogeneously in the space. The increase in the RH is directly proportional to the amount of disinfectant in the air. Because air with a lower RH readily absorbs moisture, the produced mist will move to these locations. This means that the atomized liquid, and thus the disinfectant, reaches every nook and cranny of a space (natural diffusion process). By maintaining the level of the RH for a determined period (for instance between about 30 and 120 minutes, for instance about 90 minutes) there is more than sufficient contact time between the disinfectant and the micro-organisms to eliminate these latter (decontamination process). It has been found according to the present invention that, by once again dehumidifying the air, i.e. again reducing the relative humidity, prior to and/or during atomizing of the disinfecting liquid in the air, wherein the relative humidity of the air thus increases, the concentration of the disinfecting liquid in the space decreases less quickly than without dehumidification of the air. It has been found, surprisingly, that the above described tailing effect does not occur, or hardly so, and that a considerably higher average concentration of the disinfecting liquid in the air is achieved.

According to the present invention, micro-organisms are understood to mean for instance bacteria, spores, fungi and/or viruses. The method according to the present invention is particularly effective in eliminating one or more of the micro-organisms chosen from the group consisting of *Staphylococcus aureus, MRSA, Enterococcus faecalis, Acinetobacter baumanni, Pseudomonas aeruginosa, Bacillus subtilis, Bacillus stearothermophilus, Aspergillus niger, Candida albicans, Enterococcus hirae, Mycobacterium terrae, Mycobacterium avium* and *Dermatophagoides pteronyssinus.*

The disinfecting liquid atomized according to the method preferably comprises hydrogen peroxide as disinfectant. Hydrogen peroxide is normally mixed with water and has very good disinfecting properties. It is known that the cell membranes of the micro-organisms burst open due to the oxidizing action of hydrogen peroxide. Hydrogen peroxide moreover has the advantage of breaking down into water and oxygen, and no toxic residues therefore remain. With the method according to the invention low and safe concentrations of the hydrogen peroxide can be used. A 5% solution of hydrogen peroxide in atomized form, although causing slight irritation to the mucous membranes in some cases, is completely non-hazardous in contact with humans. It has surprisingly been demonstrated according to the present invention that hydrogen peroxide in atomized form is very effective, even at low concentrations, in disinfecting spaces and/or the objects present therein.

In a particular embodiment the disinfecting liquid comprises a combination of hydrogen peroxide and silver ions. The silver ions can be present in a concentration of about 50 ppm, but may also be present in higher concentrations, for instance up to 500 ppm or more. A synergistic decontaminating effect is achieved by combining these constituents. All pathogenic bacteria, as well as the spores of for instance C. difficile, also the antibioticresistant MRSA bacteria, Acinetobacter, and viruses such as HIV, norovirus and influenza virus and fungi can, among others, be eliminated.

Using the method according to the invention, and particularly when a combination of hydrogen peroxide and silver ions is applied, lower concentrations of hydrogen peroxide can be used than is usual with the existing disinfection methods. The concentration of hydrogen peroxide in the disinfecting liquid preferably lies between 1-30%, more preferably between 1-10%, and is most preferably about 5%. This concentration gives an excellent decontaminating effect, while harmfulness and toxicity are negligible. With moderate ventilation of the space after atomization the residue that is present is already less than 1/10 of the permitted MAC (maximum acceptable concentration) value after about one hour. The MAC of a gas, vapour, mist or substance is that concentration in the air which, as far as present knowledge extends, generally does not adversely affect the health of employees or their progeny in the case of repeated exposure of an employee for a longer period or even for their working life. This applies under the following conditions:
1. for healthy adult persons;
2. for eight-hour working periods interrupted by breaks in a non-contaminated atmosphere;
3. for a working week of a maximum of 40 hours;
4. in the case of work which is physically not too heavy;
5. extra protection is available in the case of substances easily absorbed via the skin;
6. absence of other toxic substances in the workspace.
The list of MAC values forms part of schedule 3 of the policy guidelines of the Working Conditions Act in the Netherlands.

With the method according to the invention the disinfecting liquid is preferably atomized to a droplet size smaller than 10 pm, more preferably smaller than 5 µm and most preferably to a droplet size of about 1 µm. Because these very small droplets are easily taken up into the air, the disinfecting liquid is distributed very uniformly through the space and in this way reaches all surfaces of the objects possibly present in the space. It is moreover guaranteed that the disinfecting liquid together with the air reaches locations which cannot be reached with the existing disinfection techniques, such as in equipment for example. Owing to the very small droplets condensation occurs only at the saturation point.

The method according to the invention is particularly suitable for use in spaces in the medical and pharmaceutical world, such as for instance for the purpose of treating and disinfecting spaces and materials in hospitals. The method can however also be used for diverse other applications where it is desirable to reduce the number of micro-organisms, such as for instance for the purpose of disinfecting sheds in the poultry and cattle-farming sector, but also in the food industry as well as saunas and swimming pools.

The application also relates to a device for performing the above described method.

The application particularly relates to a device for disinfecting a space and the objects possibly present therein, which device at least comprises:
(a) means for determining the relative humidity of the air in the space;
(b) means for atomizing a disinfecting liquid in the space;
(c) means for monitoring and maintaining a predetermined relative humidity of the air in the space; and
(d) means for decreasing the relative humidity of the air in the space.

The means for atomizing a disinfecting liquid can comprise any suitable atomizing means, for instance ultrasonic means, an atomizing nozzle or means using sublimation gasification techniques.

The means for decreasing the relative humidity can comprise conventional air dehumidifiers, such as for instance air-conditioning systems/air dehumidifiers operating on the condensation principle, and can be placed as separate module in the space or be integrated into one device together with for instance the means for atomizing the disinfecting liquid. It can also be possible to use freeze drying techniques to solidify the moisture in the space for decreasing the relative humidity. It is in particular advantageously when the solidifying means are formed integral with the atomizing means using sublimation gasification techniques.

With the device spaces of different dimensions, such as operating rooms but also ambulances etc., can be effectively disinfected in very suitable manner.

In a preferred embodiment the device further comprises means for supplying and guiding air from the space along the disinfecting liquid.

In a particular embodiment the device further comprises indicator means for indicating the status of the device, i.e. whether the device is switched on or off. The presence of these indicator means increases safety, since it is possible to indicate when the device is switched on and disinfecting liquid is being atomized, and no staff should therefore be present in the space. The indicator means can for instance comprise visual indicator means (such as a lamp), auditory indicator means (such as a sound signal), or a combination thereof.

The device further comprises a container for the disinfecting liquid to be atomized.

In a preferred embodiment the device comprises ultrasonic means for atomizing the disinfecting liquid. The ultrasonic means preferably comprise one or more ultrasonic elements (in particular ceramic discs) which are driven by means of an ultrasonic generator, which preferably generates ultrasonic generator sound waves with a frequency of at least 1.5 MHZ, more preferably a frequency of about 1.7 MHZ. The ultrasonic elements are preferably arranged in the disinfecting liquid so that the ultrasonic vibrations are transmitted directly to the disinfecting liquid.

In a further preferred embodiment the device comprises an atomizing nozzle and means for passing the disinfecting liquid through the nozzle. Nozzles for atomizing fluids are known and any nozzle is suitable, provided that a fine mist or spray of fluid droplets is created. Preferably, the means for passing the liquid through the nozzle comprise a compressor, for instance an electric compressor or a combustion engine based compressor. The compressor is arranged to pass the liquid under pressure from the container through the atomizing nozzle, creating a fine mist or spray of droplets of disinfecting liquid.

After measurement of the initial value of the RH in the air the atomizing is started by switching on the atomizing means. As soon as a predetermined value of the RH in the air is reached, in particular a value which is 25% higher than the initial value, this RH will be maintained at this value for a determined time, for instance about 90 minutes. Prior to atomizing and/or during atomizing of the disinfecting liquid the air in the space is dehumidified using the means for decreasing the relative humidity. The atomizing means will be switched off after the desired treatment time. As long as the device is in operation, this can be indicated using indicator means, such as for instance a lamp, so that it is apparent that no staff may enter the space.

Once the disinfecting process has been carried out, the disinfecting liquid is preferably pumped back into a closed storage container in order to enhance safety and prevent liquid contamination.

The invention is further elucidated with reference to the following figures, in which:
Figure 1 shows a preferred embodiment of the device in a space to be disinfected;
Figure 2 shows a cross-section of the device of Figure 1;
Figure 3 shows schematically an atomizing unit, designated with III in Figure 2, and;
Figures 4 - 7 show results from various tests.

As shown in the embodiment of Figure 1, a space 1, for instance an operating room, is disinfected with atomizing device 2 according to the invention. Atomizing device 2 comprises a sensor 3 for measuring the relative humidity of the air in the space, which is coupled to device 2. It will be appreciated that the coupling between device 2 and sensor 3 can also take place wirelessly, for instance using Bluetooth or other radio frequency signals. As described above, for the purpose of disinfecting the space a first value (initial value) of the relative humidity (RH) of the air in the space is first measured using sensor 3. A disinfecting liquid is subsequently atomized in the space until a predetermined second value of the relative humidity of the air in the space is reached. This predetermined RH is preferably 25% higher than the initial value. This RH is maintained for a predetermined time (preferably 90 minutes) by means of atomizing the disinfecting liquid. Atomizing of the disinfecting liquid takes place by means of ultrasonic sound waves as set forth below. Prior to and/or during atomizing of the disinfecting liquid in the air, wherein the relative humidity in the air thus increases, the humidity of the air is decreased again using an air dehumidifier, such as for instance an air-conditioner on the basis of the condensation principle. Dehumidifying of the air is preferable started with a determined delay (for instance 5 to 15 minutes) after beginning atomizing of the disinfecting liquid.

As shown further in Figure 1, atomizing device 2 comprises an inlet 4 for introducing a container with the disinfecting liquid to be atomized. The device comprises two outlets 5 for the atomized disinfecting liquid 6. In the embodiment shown in Figure 1, device 2 further comprises a storage drawer 7 in which for instance loose components such as sensor 3 for measuring the RH can be stored. Device 2 further comprises indicator means consisting of one or more lamps 8 with which it is possible to indicate whether the device is switched on or off. Sensor 3 and lamps 8 are arranged on a stand. Indicator means 8 are connected to the device (this is optionally also possible via a wireless connection), and are preferably placed a distance from device 2 and more preferably outside the space 1 to be disinfected, so that it is possible to see from outside the space whether the device is switched on and disinfecting liquid is being atomized. The atomizing device as shown in Figure 1 is easy to displace to another space for disinfecting due to the presence of wheels 9 and handle 10.

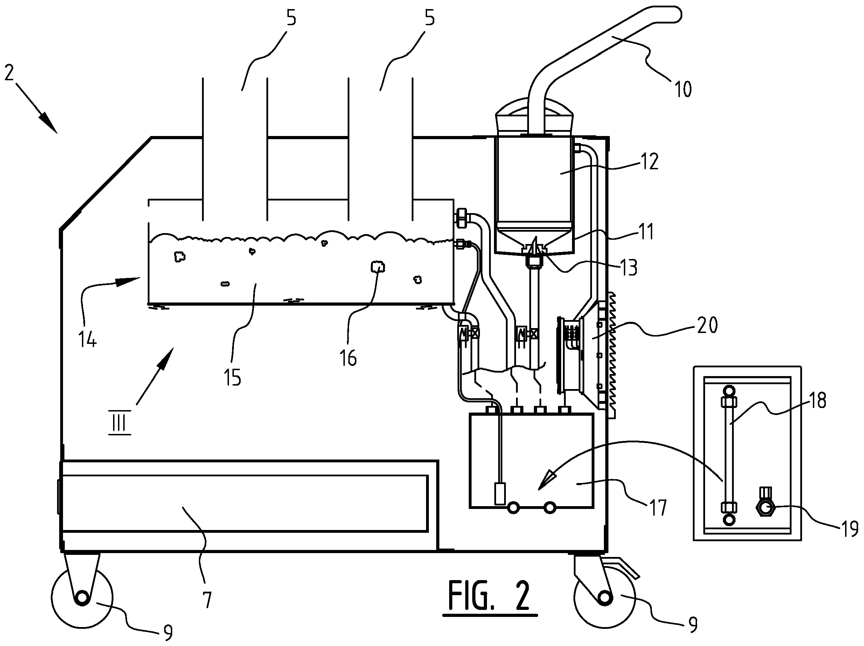

As shown in the cross-section of Figure 2, atomizing device 2 comprises in a preferred embodiment a holder 11 for receiving a container 12 with disinfecting liquid. Container 12 is arranged in holder 11 with the filling opening downward and pressed well down until filling pipe 13 protrudes through the closure, for instance a rubber cap, of the container. Storage tank 17 and atomizing unit 14 are then filled with disinfecting liquid 15. When storage tank 17 and atomizing unit 14 are filled with a minimum required quantity of disinfecting liquid 15, device 2 is ready for use. Atomizing unit 14 is in liquid communication with a storage tank 17. A gauge glass 18 on the outside of the device indicates the amount of disinfecting liquid in storage tank 17.

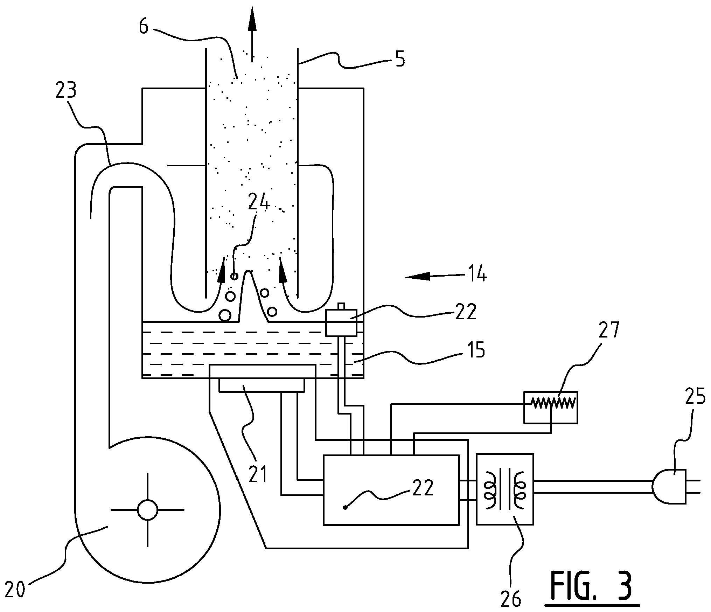

Once the initial value of the relative humidity of the air in the space has been determined and optionally also the temperature in the space, the disinfecting liquid 15 in atomizing unit 14 is set into vibration using ceramic elements as shown in Figure 3.

Figure 3 shows atomizing unit 14 schematically. Ceramic element 21 is set into vibration using ultrasonic generator 22. Figure 3 shows one ceramic element 21. The device preferably comprises a plurality of ceramic elements. Ultrasonic element 21 is in direct contact with the disinfecting liquid 15. In the shown embodiment the liquid column preferably lies between 35 and 40 mm above ultrasonic element 21. This liquid level is monitored using level switch 22. The ultrasonic elements can break down if there is no liquid thereon.

The vibration of the ultrasonic elements creates small vacuum bubbles 16 in liquid 15 (see Figure 2). These bubbles 16 subsequently implode under the influence of the surface tension of the liquid, wherein very great forces can occur, whereby the liquid is pushed out of the liquid surface in the form of very small droplets 24 (Figure 3). These small droplets are then taken up into the air and guided via outlet 5 into the space.

As shown in Figure 3, device 2 comprises a fan 20 with which air 23 is drawn out of the space into atomizing device 2 and guided through atomizing unit 14 along the surface of disinfecting liquid 15, and is then discharged via outlet pipe 5. A part of the indrawn air is preferably also blown directly to the outside through an opening around the outlet pipe. This gives a venturi effect, whereby the mist does not linger in device 2 but is, as it were, drawn out of outlet pipes 5.

The device is for instance connected to the mains electricity using plug 25. Device 2 further comprises other components such as transformer 26 and control means 27 for regulating the mist formation.

As further shown in Figure 2, the device can optionally also comprise a discharge 19 for emptying storage tank 17 if desired, for instance for servicing of the device. It is not necessary however to empty the storage tank after each use.

The present invention is not limited to the embodiment shown, but extends also to other embodiments falling within the scope of the appended claims. It should therefore be noted that the invention is not limited to the use of ultrasonic means for atomizing the disinfecting fluid. The device can therefore comprise any suitable means for atomizing the disinfecting liquid.

The effectiveness of the method and the device is illustrated in the following examples, wherein different types of hydrogen peroxide are used as disinfecting liquid.

### EXAMPLES

### EXAMPLE 1

The tests in Example 1 were performed with disinfecting liquid A comprising hydrogen peroxide in a concentration of 5% and < 0.01% silver ions. The method according to the present invention was here compared to the method as described in WO 2007/125100, i.e. the method without the step of dehumidifying the air prior to and/or during atomizing of hydrogen peroxide in the space.

### Test product A, 5% hydrogen peroxide solution in water, aerosol made using the method and device according to the invention (IC-4 principle)

### Test 1:

Test 1 performed **without** air dehumidifier.
Duration of the test: 4 hours (from 13.00 to 17.00 hours)
Initial RH 44.5%; Temperature 21.5 degrees C.
ΔRH set to 30%
The IC-4 always switches off at 76.5% RH and switches back on again at 74.% RH

### Test 2:

Test 1 performed **with** air dehumidifier. Switched on simultaneously with IC-4™
Duration of the test: 3 hours (from 10.00 to 13.00 hours)
Initial RH 44.0%; Temperature 20.5 degrees C.
ΔRH set to 30%
The IC-4 always switches off at 75.5% RH and switches back on again at 73.% RH

### Comparison of Test 1 and Test 2 of Product A with 5% hydrogen peroxide solution in water

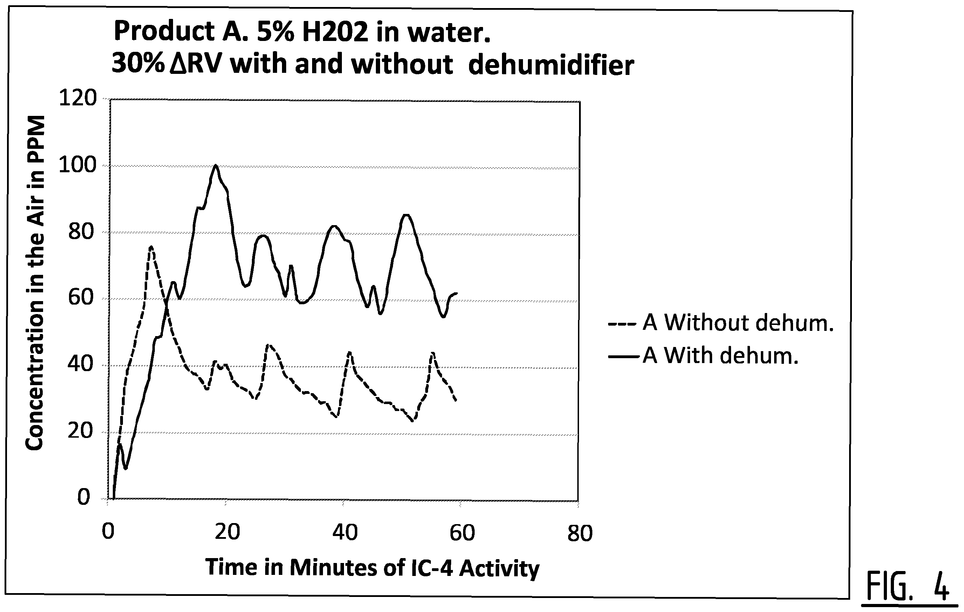

In figure 4 the combined results from Test 1 without and test 2 with dehumidifier are shown.

| | Without Air Dehumidifier | With Air Dehumidifier |
|---|---|---|
| Average Concentration H2O2: | 33 ppm | **66 ppm** |
| **Difference: 100% and no "tailing"** | | |

Difference: more rapid rise to maximum concentration without air dehumidifier, but average much lower.

### EXAMPLE 2

The tests in Example 2 were performed with disinfecting liquid B comprising hydrogen peroxide in a concentration of 4.9% and < 0,01% silver ions. The method according to the present invention was again compared to the method as described in WO 2007/125100, i.e. the method without the step of dehumidifying the air prior to and/or during atomizing of the hydrogen peroxide in the space.

### Test Product B, 4.9% hydrogen peroxide solution in water, aerosol made using IC-4 principle

### Test 1:

Test 1 performed **without** air dehumidifier.
Duration of the test: from 12.00 to 14.00 hours
Initial RH 45%; Temperature 21.5 degrees C.
ΔRH set to 30%
The IC-4 always switches off at 76.5% RH and switches back on again at 74.% RH

### Test 2:

Test 2 performed **with** air dehumidifier. Switched on simultaneously with IC-4™
Duration of the test: from 12.00 to 14.00 hours
Initial RH 44.0%; Temperature 20.5 degrees C.
ΔRH set to 30%
The IC-4 always switches off at 75.5% RH and switches back on again at 73.% RH

### Comparison of Test 1 and Test 2 Product B 4.9% hydrogen peroxide solution in water

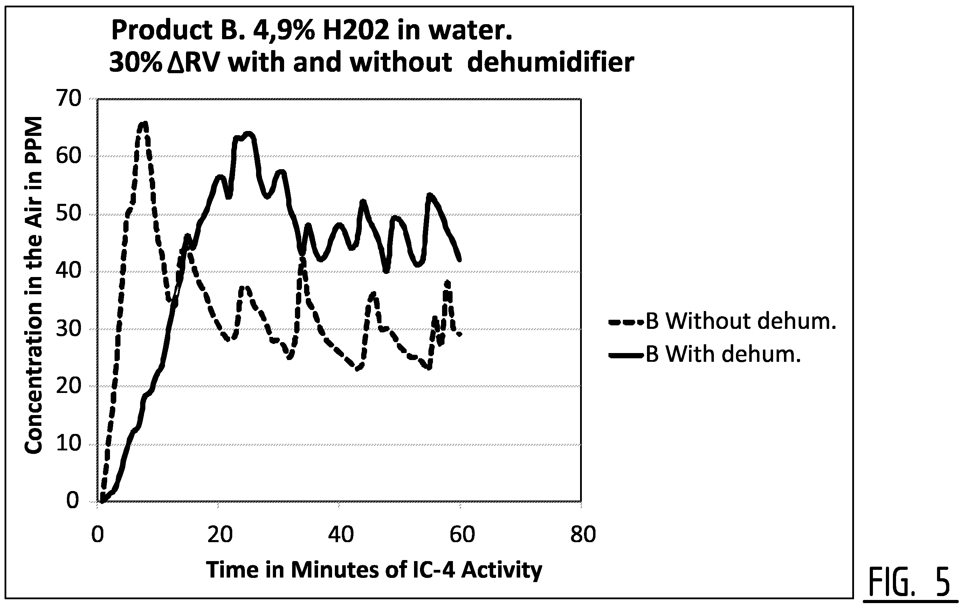

In figure 5 the combined results from Test 1 without and Test 2 with dehumidifier are shown.

| | Without Air Dehumidifier | With Air Dehumidifier |
|---|---|---|
| Average Concentration H2O2: | 29.63 ppm | 44.48 ppm |
| **Difference: 50% and no "tailing"** | | |

Difference: more rapid rise to maximum concentration without air dehumidifier, but average much lower.

### EXAMPLE 3

The tests in Example 3 were performed with disinfecting liquid C comprising hydrogen peroxide in a concentration of 5% without silver ions. The method according to the present invention was again compared to the method as described in WO 2007/125100, i.e. the method without the step of dehumidifying the air prior to and/or during atomizing of hydrogen peroxide in the space.

### Test Product C, 5% hydrogen peroxide solution in water, aerosol made using IC-4 principle

### Test 1 (without air dehumidifier):

Test 1 performed **without** air dehumidifier.
Duration of the test: 11.15 to 13.15 hours
Initial RH 45%; Temperature 22 degrees C.
ΔRH set to 30%
The IC-4 always switches off at 76.5% RH and switches back on again at 74.5% RH

### Test 2:

Test performed **with** air dehumidifier.
Duration of test: 14.35 to 16.35 hours
Initial RH 45%; Temperature 22 degrees C.
ΔRH set to 30%
The IC-4 always switches off at 76.5% RH and switches back on again at 74.5% RH

### Comparison of Test 1 and Test 2 Product C, 5% hydrogen peroxide solution in water

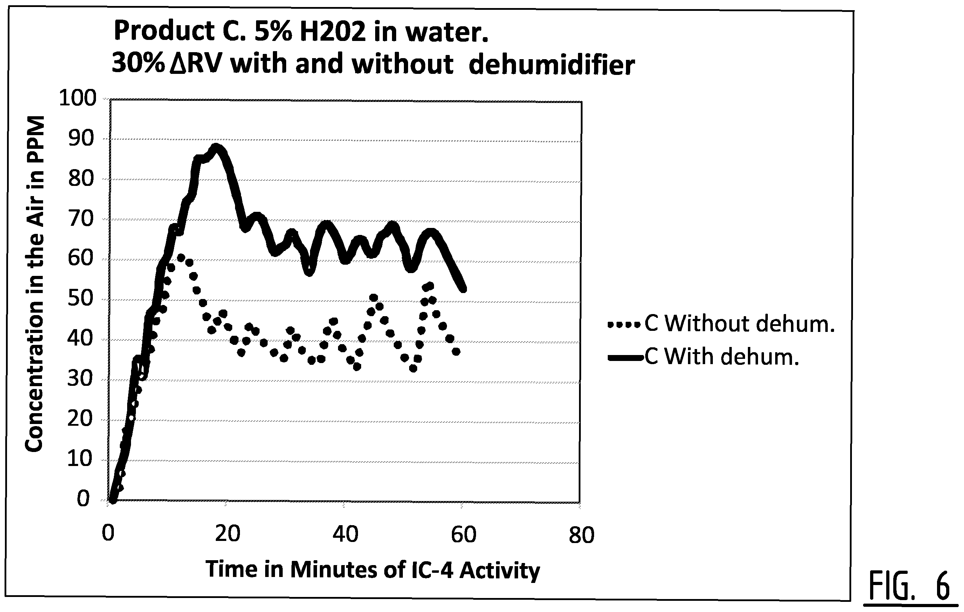

In figure 6 the combined results from Test 1 without and Test 2 with dehumidifier are shown.

| | Without Air Dehumidifier | With Air Dehumidifier |
|---|---|---|
| Average Concentration H2O2: | 35.53 ppm | 62.23 ppm |

### Difference: 75% and practically no "tailing"

Difference: more rapid rise to maximum concentration without air dehumidifier, but average much lower.

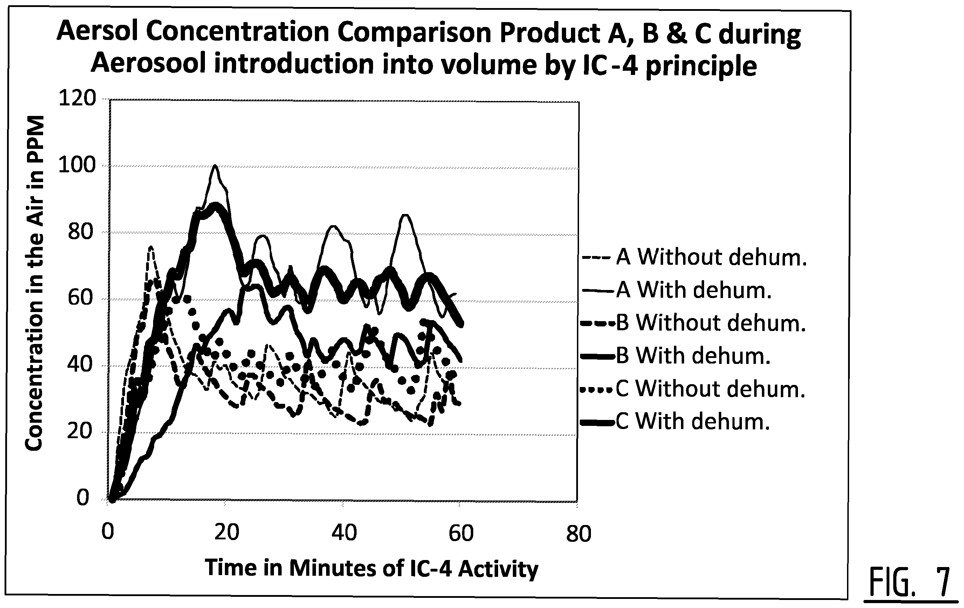

In figure 7 a comparison of the disinfecting liquids is shown.

The foregoing Examples clearly show that with the method and device much higher concentrations of disinfecting liquid can be maintained during the disinfection treatment than without the step of dehumidifying the air. The method is hereby more effective and it is for instance possible to operate with lower concentrations of disinfecting liquid and/or shorter treatment times.

## Claims

1. Method for disinfecting a space and the objects present therein, which method comprises at least the following steps of:
(a) determining a first value of the relative humidity of the air in the space;
(b) atomizing a disinfecting liquid in the space until a predetermined second value of the relative humidity of the air is reached in the space, and;
(c) maintaining the relative humidity of the air for a predetermined time at the second value by means of atomizing the disinfecting liquid,
wherein the method further comprises extracting moisture from the air in the space during atomizing of the disinfecting liquid.

2. Method as claimed in claim 1, wherein the extraction of moisture from the air in the space is started with a predetermined delay relative to starting of the atomization of the disinfecting liquid, wherein the predetermined delay preferably comprises 1-15 minutes, more preferably 5-10 minutes.

3. Method as claimed in claim 1 or 2, wherein the extraction of moisture from the air is carried out continuously during atomizing of the disinfecting liquid.

4. Method as claimed in any of the claims 1-3, wherein atomizing of the disinfecting liquid takes place by means of ultrasonic sound waves, preferably at a frequency of 1.5 MHz - 2.8 MHz, more preferably at a frequency of 1.5 MHz - 2.0 MHz and most preferably at a frequency of 1.7 MHz.

5. Method as claimed in any of the claims 1-4, wherein the atomizing of the disinfecting liquid takes place by passing the disinfecting liquid through a nozzle, in particular an atomizing nozzle, and/or by sublimation gasification.

6. Method as claimed in any of the claims 1-5, wherein the second value of the relative humidity is higher than the first value, wherein the second value is preferably at least 10% higher, more preferably at least 15% higher, and even more preferably at least 20% higher than the first value, and most preferably the second value is 25% higher than the first value.

7. Method as claimed in any of the claims 1-6, wherein the disinfecting liquid comprises hydrogen peroxide.

8. Method as claimed in claim 7, wherein the disinfecting liquid comprises a combination of hydrogen peroxide and silver ions.

9. Method as claimed in any of the claims 1-8, wherein the disinfecting liquid is atomized to a droplet size smaller than 10 pm, preferably smaller than 5 µm and more preferably the droplet size is 1 µm.

## Patentansprüche

1. Verfahren zur Desinfektion eines Raumes und der darin enthaltenen Objekte, umfassend mindestens die folgenden Schritte:
(a) Bestimmung eines ersten relativen Luftfeuchtigkeitswertes in dem Raum;
(b) Zerstäubung einer Desinfektionsflüssigkeit in dem Raum, bis ein vorbestimmter zweiter relativer Luftfeuchtigkeitswert in dem Raum erreicht ist, und
(c) Aufrechterhaltung der relativen Luftfeuchtigkeit über einen vorbestimmten Zeitraum auf dem zweiten Wert mittels Zerstäubung der Desinfektionsflüssigkeit,
wobei das Verfahren weiterhin die Extraktion von Feuchtigkeit aus der Luft in dem Raum während der Zerstäubung des Desinfektionsmittels umfasst.

2. Verfahren gemäß Anspruch 1, wobei die Extraktion der Feuchtigkeit aus der Luft in dem Raum mit einer vorbestimmten Verzögerung im Verhältnis zum Beginn der Zerstäubung der Desinfektionsflüssigkeit begonnen wird, wobei die vorbestimmte Verzögerung vorzugsweise 1 bis 15 Minuten, besonders bevorzugt 5 bis 10 Minuten, beträgt.

3. Verfahren gemäß Anspruch 1 oder 2, wobei die Extraktion der Feuchtigkeit aus der Luft kontinuierlich während der Zerstäubung der Desinfektionsflüssigkeit durchgeführt wird.

4. Verfahren gemäß einem der Ansprüche 1-3, wobei die Zerstäubung der Desinfektionsflüssigkeit mittels Ultraschallwellen stattfindet, bevorzugt bei einer Frequenz von 1,5 MHz - 2,8 MHz, besonders bevorzugt bei einer Frequenz von 1,5 - 2 MHz, und meist bevorzugt bei einer Frequenz von 1,7 MHz.

5. Verfahren gemäß einem der Ansprüche 1 - 4, wobei die Zerstäubung der Desinfektionsflüssigkeit mittels Durchleitung der Desinfektionsflüssigkeit durch eine Düse, insbesondere eine Zerstäubungsdüse, und/oder durch Sublimationsvergasung stattfindet.

6. Verfahren gemäß einem der Ansprüche 1 - 5, wobei der zweite Wert der relativen Feuchtigkeit höher ist als der erste Wert, wobei der zweite Wert vorzugsweise mindestens 10% höher, besonders bevorzugt mindestens 15% höher und noch bevorzugter mindestens 20% höher als der erste Wert und der zweite Wert meist bevorzugt 25% höher als der erste Wert ist.

7. Verfahren gemäß einem der Ansprüche 1 - 6, wobei die Desinfektionsflüssigkeit Wasserstoffperoxid enthält.

8. Verfahren gemäß Anspruch 7, wobei die Desinfektionsflüssigkeit eine Kombination von Wasserstoffperoxid und Silberionen enthält.

9. Verfahren gemäß einem der Ansprüche 1 - 8, wobei die Desinfektionsflüssigkeit zu einer Tröpfchengröße kleiner als 10 µm, bevorzugt kleiner als 5 µm und meist bevorzugt einer Tröpfchengröße von 1 µm zerstäubt wird.

## Revendications

1. Procédé de désinfection d'un espace et des objets présents à l'intérieur, lequel procédé comprend au moins les étapes suivantes :
(a) détermination d'une première valeur de l'humidité relative de l'air dans l'espace ;
(b) atomisation d'un liquide désinfectant dans l'espace jusqu'à ce qu'une seconde valeur prédéterminée de l'humidité relative de l'air soit atteinte dans l'espace, et
(c) maintien de l'humidité relative de l'air pendant une durée prédéterminée à la seconde valeur par atomisation du liquide désinfectant,
dans lequel le procédé comprend en outre l'extraction d'humidité de l'air dans l'espace au cours de l'atomisation du liquide désinfectant.

2. Procédé selon la revendication 1, dans lequel l'extraction d'humidité de l'air dans l'espace démarre avec un retard prédéterminé par rapport au démarrage de l'atomisation du liquide désinfectant, dans lequel le retard prédéterminé comprend de préférence 1 à 15 minutes, de manière davantage préférée, 5 à 10 minutes.

3. Procédé selon la revendication 1 ou 2, dans lequel l'extraction d'humidité de l'air est réalisée en continu au cours de l'atomisation du liquide désinfectant.

4. Procédé selon l'une quelconque des revendications 1 à 3, dans lequel l'atomisation du liquide désinfectant se produit au moyen d'ondes ultrasonores, de préférence à une fréquence de 1,5 MHz à 2,8 MHz, de manière davantage préférée, à une fréquence de 1,5 MHz à 2,0 MHz et de manière préférée entre toutes, à une fréquence de 1,7 MHz.

5. Procédé selon l'une quelconque des revendications 1 à 4, dans lequel l'atomisation du liquide désinfectant se produit par le passage du liquide désinfectant à travers une buse, en particulier une buse d'atomisation, et/ou par gazéification-sublimation.

6. Procédé selon l'une quelconque des revendications 1 à 5, dans lequel la seconde valeur de l'humidité relative est supérieure à la première valeur, dans lequel la seconde valeur est de préférence au moins de 10 % supérieure, de manière davantage préférée au moins de 15 % supérieure, et de manière encore davantage préférée d'au moins 20 % supérieure à la première valeur, et de manière préférée entre toutes, la seconde valeur est de 25 % supérieure à la première valeur.

7. Procédé selon l'une quelconque des revendications 1 à 6, dans lequel le liquide désinfectant comprend du peroxyde d'hydrogène.

8. Procédé selon la revendication 7, dans lequel le liquide désinfectant comprend une combinaison de peroxyde d'hydrogène et d'ions d'argent.

9. Procédé selon l'une des revendications 1 à 8, dans lequel le liquide désinfectant est atomisé à une taille de gouttelette inférieure à 10 µm, de préférence inférieure à 5 µm et de manière davantage préférée, la taille de la gouttelette est de 1 µm.
